# EUROPEAN PATENT APPLICATION

(11) **EP 2 962 642 A1**
(43) Date of publication of application: **06.01.2016**
(21) Application number: 13876474.1
(22) Date of filing: 28.02.2013
(51) Int. Cl.: A61B 8/14, A61N 7/00

(54) **METHOD FOR DETECTING CAVITATION AND ULTRASONIC MEDICAL APPARATUS THEREFOR**

(71) Applicant: Alpinion Medical Systems Co., Ltd., Hwaseong-si, Gyeonggi-do 445-380 (KR)
(72) Inventor: SON, Keonho, Seongnam-si Bundang-gu Gyeonggi-do 463-440 (KR); KANG, Kookjin, Yongin-si Gyeonggi-do 448-536 (KR); KIM, Dae Seung, Seoul 157-882 (KR); JUN, Sukhwan, Incheon 402-200 (KR)
(74) Representative: Thorniley, Peter
(86) International application number: PCT/KR2013/001670
(87) International publication number: WO 2014/133209

(57) **Abstract**

A method for detecting a cavitation and an ultrasound medical apparatus for implementing the method are disclosed. A method for detecting a cavitation and an ultrasound medical apparatus for focusing an imaging ultrasound and a high-intensity ultrasound on a target object (an effected area of a patient), processing a reflected signal from the target object, and when it is determined that a cavitation is developed, detecting a location of the cavitation based on a data processing of the reflected signal.

## Description

### [Technical Field]

The present disclosure relates to a method for detecting a cavitation and an ultrasound medical apparatus therefor, and more particularly, to a method for detecting a location where a cavitation is developed by focusing an imaging ultrasound and a high-intensity ultrasound on a target object (an effected area of a patient) and processing a reflected signal therefrom and an ultrasound medical apparatus for implementing the method.

### [Background]

The statements in this section merely provide background information related to the present disclosure and do not necessarily constitute prior art.

A high-intensity focused ultrasound is generally used for treating (processing) a biological tissue such as cancer, tumor, and lesion. The treatment modality using the high-intensity ultrasound is a method for transmitting a high-intensity ultrasound by focusing it on a single area and necrosing the corresponding biological tissue by using a heat generated by the high-intensity ultrasound. In this case, damage on a healthy biological tissue by the high-intensity ultrasound should be avoided. The treatment (processing) using the high-intensity ultrasound can eliminate an incision from a surgery.

In the treatment modality using the high-intensity ultrasound, an ultrasound for acquiring an image is transmitted to a biological tissue to be treated and a high-intensity ultrasound is transmitted to the corresponding biological tissue after acquiring the image by using an echo signal reflected from the tissue; however, this may cause a tissue destruction phenomenon due to a cavitation caused by the high-intensity ultrasound. The cavitation is a phenomenon in which, when the biological tissue is exposed to the high-intensity ultrasound, moisture in a cell is transformed into gaseous phase by a low pressure due to a negative part, thus generating a microbubble, the microbubble is increased in size to cause a resonance and eventually bursts to generate a high-pressure shock wave, thus destructing the surrounding biological tissues. However, the location of such a cavitation is hard to detect in a precise manner, and hence the biological tissue out of a treatment area may not be prevented from being damaged.

### [Disclosure]

### [Technical Problem]

The present disclosure has been made in an effort to effectively resolve the above-mentioned problems, and it is an object of some embodiments of the present disclosure to provide a method for detecting a cavitation and an ultrasound medical apparatus for focusing an imaging ultrasound and a high-intensity ultrasound on a target object (an affected area of a patient) and detecting the location of a cavitation by processing a reflected signal from the target object.

### [Summary]

According to some embodiments of the present disclosure, an ultrasound medical apparatus is provided, which includes an imaging transducer configured to transmit an imaging ultrasound to a target object and to receive a first echo signal reflected from the target object, a treatment transducer configured to transmit a high-intensity ultrasound to a focal-point spot on the target object, a data processing unit configured to receive a second echo signal reflected from the focal-point spot, to divide scanline data acquired from the second echo signal in a time-division manner, to generate time-division data, to convert the time-division data into frequency domain data, and to generate result data from the frequency domain data, and a detecting unit configured to detect a location of a cavitation based on a frequency component included in the result data.

Another embodiment of the present disclosure provides a method for detecting a cavitation by an ultrasound medical apparatus, which includes a first echo signal receiving step, a high-intensity ultrasound transmitting step, a data processing step and a detecting step. The first echo signal receiving step includes transmitting an imaging ultrasound to a target object, and receiving a first echo signal reflected from the target object. The high-intensity ultrasound transmitting step includes transmitting a high-intensity ultrasound to a focal-point spot on the target object. The data processing step includes dividing scanline data acquired from a second echo signal reflected from the focal-point spot in a time-division manner and generating time-division data, converting the time-division data into frequency domain data, and generating result data from the frequency domain data. The detecting step includes detecting a location of a cavitation based on a frequency component included in the result data.

### [Advantageous Effects]

According to some embodiments of the present disclosure as described above, developed amount and location of a cavitation can be detected by focusing an imaging ultrasound and a high-intensity ultrasound on a target object (an affected area of a patient) and processing a reflected signal from the target object, and a stable treatment of a lesion and a temperature measurement can be performed. Further, according to some embodiments, the location of a cavitation developed in real time can be found when performing a treatment by using a high-intensity ultrasound, and hence a delay of the treatment due to the cavitation can be avoided.

Further, according to some embodiments, the duration time for transmitting the high-intensity ultrasound to the target object can be increased by detecting the location of the cavitation, and hence the time for treating a biological tissue can be shortened. Moreover, according to some embodiments, a biological tissue other than the treatment area can be prevented from being damaged, by precisely detecting the location of the cavitation.

### [Brief Description of Drawings]

FIG. 1 is a schematic block diagram of an ultrasound medical apparatus according to some embodiments of the present disclosure.
FIG. 2 is a schematic block diagram of a cavitation detecting unit included in an ultrasound medical apparatus according to some embodiments.
FIG. 3 is a flowchart of a method for detecting a cavitation in an ultrasound medical apparatus according to some embodiments.
FIG. 4 is a schematic diagram of an operation of transmitting and receiving an imaging ultrasound and a treatment ultrasound by an ultrasound medical apparatus according to some embodiments.
FIG. 5 is a graph of a first echo signal and a second echo signal according to some embodiments.
FIG. 6 is a graph of an operation of processing data for detecting a cavitation in an ultrasound medical apparatus according to some embodiments.
FIG. 7 is a graph of an operation of dividing result data by magnitude and calculating respective summing result values in an ultrasound medical apparatus according to some embodiments.

**REFERENCE NUMERALS**

| | | | |
|---|---|---|---|
| 100: | Ultrasound Medical Apparatus | 110: | Imaging Transducer |
| 120: | Treatment Transducer | 122: | Synchronizing Unit |
| 130: | Image Processing Unit | 140: | Cavitation Detecting Unit |
| 142: | Data Processing Unit | 144: | Detecting Unit |
| 150: | User Input Unit | 160: | Display Unit |
| 170: | Storage Unit | 210: | Data Acquiring Unit |
| 220: | Data Converting Unit | 230: | Filter Processing Unit |
| 240: | Summing Processing Unit | 250: | Comparing Unit |
| 260: | Mapping Unit | | |

### [Detailed Description]

Hereinafter, at least one embodiment of the present disclosure will be described in detail with reference to the accompanying drawings.

A diagnostic image described in some embodiments includes a B-mode image, a C-mode image, and the like. The B-mode image is a grayscale image in an image mode for displaying a motion of a target object, and the C-mode image is an image in a color flow image mode. A BC-mode image is an image in an image mode for displaying a blood flow or a motion of the target object by using a Doppler effect, which simultaneously provides the B-mode image and the C-mode image to provide anatomic information together with the blood flow and the motion of the target object. That is, the B-mode is a grayscale image mode for displaying the motion of the target object, and the C-mode is a color flow image mode for displaying the blood flow or the motion of the target object. An ultrasound medical apparatus 100 according to some embodiments is capable of simultaneously providing a B-mode image and the C-mode image that is a color flow image; however, for the sake of convenience in explanation, descriptions are given with an assumption that a diagnostic image provided by the ultrasound medical apparatus 100 in the present disclosure is a B-mode image.

In some embodiments, a first echo signal means an echo signal obtained by receiving a transmitted imaging ultrasound after being reflected at a target object, and a second echo signal means an echo signal obtained by receiving an imaging ultrasound and a high-intensity ultrasound after they reached a target object at the same time and reflected at the target object.

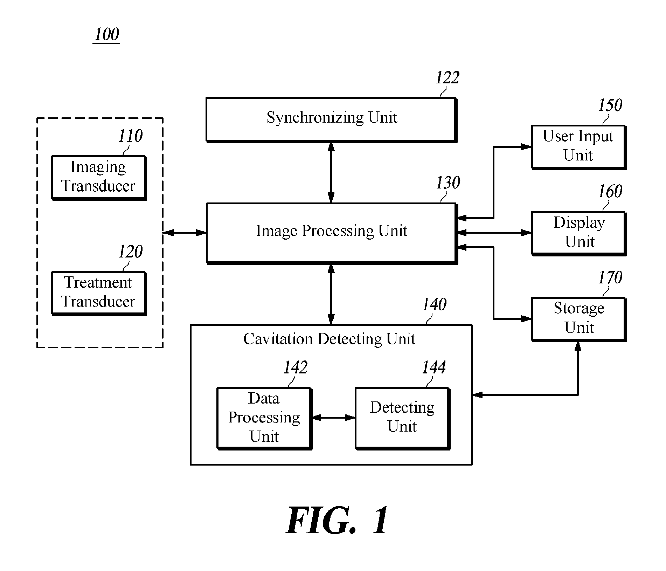

FIG. 1 is a block diagram of an ultrasound medical apparatus according to some embodiments.

The ultrasound medical apparatus 100 according to some embodiments includes an imaging transducer 110, a treatment transducer 120, a synchronizing unit 122, an image processing unit 130, a cavitation detecting unit 140, a user input unit 150, a display unit 160, and a storage unit 170. These specified components of the ultrasound medical apparatus 100 according to some embodiments are merely exemplary for describing the technical idea of some embodiments, although one of ordinary skill would appreciate that various modifications, additions and substitutions are possible in the constituent elements of the ultrasound medical apparatus 100 without departing from the spirit and scope of the embodiments.

The imaging transducer 110 includes an imaging transducer array, transmits an imaging ultrasound to a target object, and receives a first echo signal reflected at the target object. The transducer converts an electrical analog signal into an imaging ultrasound signal, transmits the imaging ultrasound signal to the target object, converts the first echo signal reflected at the target object into an electrical analog signal, and includes a plurality of transducer elements coupled to each other. The imaging transducer 110 transmits a focused ultrasound beam to the target object along a scanline by appropriately delaying an input time of a pulse that is inputted to each transducer. The first echo signal reflected at the target object is inputted to each transducer with a different reception time, and each transducer outputs the inputted first echo signal to a beamformer (not shown).

The imaging transducer 110 operates in a manner that the imaging ultrasound is transmitted to the target object, and the first echo signal reflected at the target object is received to form a reception signal. Further, when the treatment transducer 120 transmits a high-intensity ultrasound to a focal-point spot (a spot corresponding to focal-point information within a region of interest set on the target object) of the region of interest (ROI), the imaging transducer 110 receives a second echo signal reflected at the target object corresponding to the imaging ultrasound and the high-intensity ultrasound. The second echo signal means an echo signal obtained by receiving the imaging ultrasound and the high-intensity ultrasound after they reached the target object at the same time and reflected at the target object.

The treatment transducer 120 includes a high-intensity transducer array, and performs an operation of transmitting a high-intensity ultrasound to the focal-point spot of the target object. The treatment transducer 120 transmits the high-intensity ultrasound to the focal-point spot (a spot corresponding to the focal-point information within the region of interest set on the target object) within a predetermined region of interest. The region of interest means a region set via the user input unit 150 within a diagnostic image formed based on the first echo signal obtained from the imaging ultrasound transmitted to the target object via the imaging transducer 110 and reflected at the target object. The focal-point spot is represented by coordinate values of a point to which the high-intensity ultrasound is transmitted via the treatment transducer 120 within the region of interest, which is a location corresponding to the focal-point information inputted from the user input unit 150. In some embodiments, the treatment transducer 120 has a circular shape, which is formed in a manner that the imaging transducer 110 is located at the center; however, the shape and structure of the treatment transducer 120 are not limited to this scheme.

The synchronizing unit 122 synchronizes times at which the imaging transducer 110 and the treatment transducer 120 generate and transmit the ultrasounds, respectively, to allow the second echo signal to be received. More specifically, the synchronizing unit 122 calculates the distance between the focal-point spot and the imaging transducer 110 based on the first echo signal reflected at the target object to generate a diagnostic image, and synchronizes the transmission times of the imaging ultrasound and the high-intensity ultrasound such that the high-intensity ultrasound and the imaging ultrasound simultaneously reach the focal-point spot.

The image processing unit 130 generates a diagnostic image by using a reception signal obtained based on an imaging ultrasound echo signal corresponding to the imaging ultrasound from the imaging transducer 110, and outputs the diagnostic image to the display unit 160. The image processing unit 130 further sets a region of interest on the diagnostic image according to an operation or instructions from the user input unit 150.

The image processing unit 130 includes a beamformer, a signal processing unit, and a scan converting unit. The beamformer focuses the first echo signal or the second echo signal received by each transducer included in the imaging transducer 110. Further, the beamformer forms a reception focused signal by adding an appropriate delay to each electrical digital signal with consideration of a time for reaching each transducer of the imaging transducer 110 and the treatment transducer 120 from the target object and then summing the digital signals.

That is, when the imaging transducer 110 transmits the imaging ultrasound or the treatment transducer 120 transmits the high-intensity ultrasound, the beamformer controls driving timing of each transducer included in the imaging transducer 110 and the treatment transducer 120 to focus the ultrasound toward the focal-point spot. Further, considering the fact that the time for the first echo signal or the second echo signal reflected at the target object to reach each transducer of the imaging transducer 110 is different, the beamformer adds a time delay to the imaging ultrasound or the high-intensity ultrasound of the imaging transducer 110 and the treatment transducer 120, and then focuses the first echo signal or the second echo signal. This beamformer includes an imaging beamformer corresponding to the imaging transducer 110 and a treatment beamformer corresponding to the treatment transducer 120. The imaging beamformer includes a transmit beamformer and a receive beamformer, and the treatment beamformer includes a transmit beamformer; however, the configurations of the imaging beamformer and the treatment beamformer are not limited to this scheme.

The signal processing unit performs a digital signal processing on the first echo signal or the second echo signal focused by the beamformer, and generates a diagnostic image. The scan converting unit converts the diagnostic image into an image of a data format used in the display unit 160 of a predetermined scanline display system. That is, the scan converting unit converts the diagnostic image signal into a signal of a data format that is actually displayed on the display unit 160.

The image processing unit 130 further outputs the region of interest for the generation location or development location of the cavitation detected by the cavitation detecting unit 140 by overlaying the region of interest on the diagnostic image (e.g., a B-mode image). At this time, the development location of the cavitation can be displayed with a different tone density depending on a comparison result value on the region of interest.

The cavitation detecting unit 140 divides data for each scanline acquired from the second echo signal with a predetermined time, to generate time-division data, and detects the location of the cavitation by using result data obtained by converting each piece of time-division data into frequency-domain data.

The cavitation detecting unit 140 includes a data processing unit 142 and a detecting unit 144.

The data processing unit 142 receives the second echo signal (the signal received by the imaging transducer 110) from the image processing unit 130, and divides the second echo signal based on a scanline to transmit the imaging ultrasound to the region of interest, so as to acquire the data for each scanline. The data for each scanline can be amplitude data. In this step of acquiring the data for each scanline, the data processing unit 142 changes the data for each scanline to data on phase components (e.g., I, Q data) to allow the data to be processed in a time-division manner; however, the present disclosure is not limited to this scheme, but the acquired data for each scanline (e.g., the amplitude data) can also be processed in a time-division manner without changing the data for each scanline to the data on the phase components.

The data processing unit 142 divides the acquired data for each scanline with a predetermined time, to generate a plurality of pieces of time-division data, converts each piece of time-division data into frequency-domain data by using a frequency analysis, and generates result data by filtering a high-intensity frequency component included in the frequency-domain data. The frequency-domain data can be obtained by using a Fast Fourier Transform (FFT) or the like. The data processing unit 142 can obtain the result data by filtering a main frequency component and a harmonic frequency component from the frequency-domain data by using a filter (sawtooth filter) having a sawtooth-shaped window or the like.

The detecting unit 144 calculates a summing result value by summing signal magnitude by frequency for each piece of result data received from the data processing unit 142, and compares the calculated summing result value with a predetermined threshold value, to generate a comparison result value. That is, the detecting unit 144 calculates the summing result value for each of a plurality of pieces of result data for the region of interest, and compares the threshold value that is set based on the first echo signal with each summing result value for each region obtained by dividing the region of interest for each scanline in a time-division manner, to generate the comparison result value. Further, the detecting unit 144 can detect the location where a cavitation is developed in the region of interest by dividing the region of interest set for the target object by the scanline, dividing each scanline in a time-division manner, and mapping the comparison result value corresponding to each time-division region.

The user input unit 150 receives instructions from an operation or an input by a user. The user instructions include a setting instruction for controlling the imaging ultrasound and the high-intensity ultrasound of the ultrasound medical apparatus 100, a setting instruction for setting coordinate values of the region of interest or the focal-point spot, and the like.

The user input unit 150 receives location-of-interest information from a user for setting the region of interest on the diagnostic image or sets the region of interest via a control of a direction key such as a joystick, a mouse, or the like. This allows the ultrasound medical apparatus 100 to transmit the high-intensity ultrasound to the region of interest of the target object, such as cancer tissue, tumor tissue, lesion tissue, and the like. Further, the user input unit 150 receives focal-point information for setting a focal-point spot to transmit the high-intensity ultrasound into the region of interest. The display unit 160 outputs an image received from the image processing unit 130 as a B-mode image or a C-mode image. The storage unit 170 is a storage means for storing various pieces of data required to drive the ultrasound medical apparatus 100, and stores a reception signal obtained based on the first echo signal, a threshold value set based on the first echo signal, and the like.

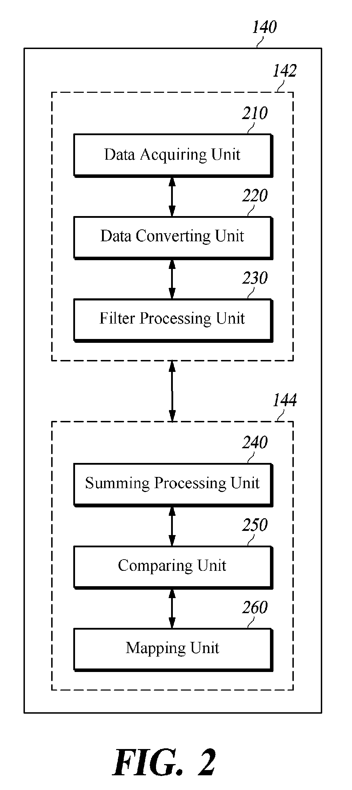

FIG. 2 is a schematic block diagram of a cavitation detecting unit included in an ultrasound medical apparatus according to some embodiments.

According to some embodiments, the cavitation detecting unit 140 includes the data processing unit 142 and the detecting unit 144. The data processing unit 142 includes a data acquiring unit 210, a data converting unit 220 and a filter processing unit 230. The detecting unit 144 includes a summing processing unit 240, a comparing unit 250 and a mapping unit 260.

The data acquiring unit 210 acquires data on the second echo signal received by the imaging transducer 110. The second echo signal means an echo signal obtained by the imaging ultrasound and the high-intensity ultrasound simultaneously after they reached the target object and reflected at the target object.

According to some embodiments, the data acquiring unit 210 receives the second echo signal from the image processing unit 130, divides the imaging ultrasound based on the scanline of region of interest, and acquires data for each scanline.

The data converting unit 220 divides the acquired data for each scanline with a predetermined time to generate a plurality of pieces of time-division data, and converts each piece of time-division data into frequency-domain data by using a frequency analysis. In some embodiments, the data are divided at regular time interval, and the regular time interval means an interval corresponding to a depth of the target object.

The data converting unit 220 converts the time-division data in a time domain into the frequency-domain data by using a frequency analysis based on the Fast Fourier Transform (FFT); however, the present disclosure is not limited to this scheme, but any type of frequency conversion, such as the Laplace Transform, can be applied so long as the time-domain data can be converted into the frequency-domain data. A method for performing the FFT is roughly an algorithm designed to decrease the number of calculations when calculating a Discrete Fourier Transform using a formula to obtain an approximate value based on the Fourier Transform. The FFT is a known conversion algorithm, and thus a detailed description thereof is omitted.

The filter processing unit 230 filters a predetermined frequency component from the frequency-domain data outputted from the data converting unit 220, to generate result data.

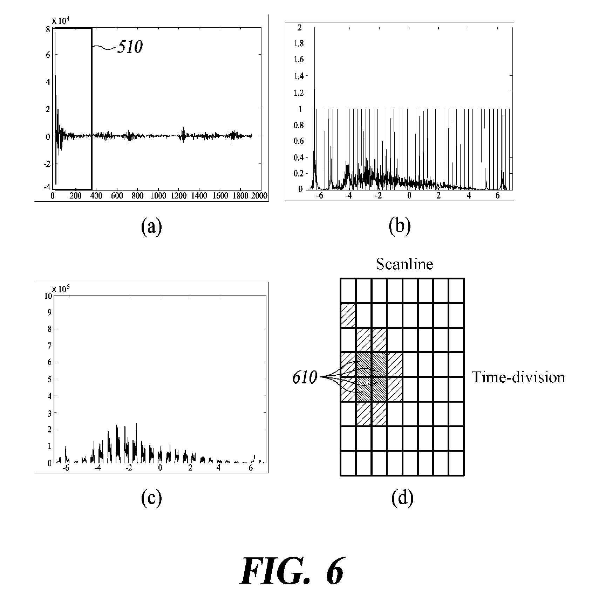

According to some embodiments, the filter processing unit 230 generates the result data by filtering a high-intensity frequency component corresponding to the high-intensity ultrasound included in the frequency-domain data. In this case, the high-intensity frequency component includes a main frequency component (e.g., a frequency of 1 MHz when the high-intensity frequency is 1 MHz) and a harmonic frequency component (a frequency of integer multiple of 1 MHz when the high-intensity frequency is 1 MHz). The main frequency component means a frequency component corresponding to the echo signal of the high-intensity ultrasound, and the harmonic frequency component means a frequency component of an integer multiple of the echo signal of the high-intensity ultrasound. The filter processing unit 230 filters the high-intensity frequency component by using a filter having a sawtooth-shaped window; however, the present disclosure is not limited to this scheme, but any type of filter can be used so long as a specific frequency component is filtered. The filter having a sawtooth-shaped window can perform the filtering in a sawtooth-shaped form shown at (b) in FIG. 6.

The filter processing unit 230 transmits the generated result data to the detecting unit 144. The result data includes a frequency component for the imaging ultrasound, a cavitation signal, and a frequency component for a noise signal.

The summing processing unit 240 sums signal magnitudes by frequency for each piece of result data received from the data processing unit 142, and outputs a summing result value. The summing processing unit 240 calculates the summing result value for each of a plurality of pieces of result data for the region of interest, and allows the development of the cavitation to be confirmed by using a plurality of summing result values obtained in this manner.

In some embodiments, the summing processing unit 240 calculates a single summing result value from a single piece of result data; however, the present disclosure is not limited to this scheme, but the result data can be divided by a frequency band, thus calculating a plurality of summing result values for each frequency band. For example, when the result data is divided by a high-frequency band and a low-frequency band and the summing result value is calculated for each frequency band, a high-frequency summing result value can be calculated by summing magnitudes of high frequency components included in the high-frequency band, and a low-frequency summing result value can be calculated by summing magnitudes of low-frequency components included in the low-frequency band. The summing processing unit 240 allows the calculated high-frequency summing result value to be compared with a predetermined threshold value, to generate a comparison result value, and allows the development location of a cavitation to be detected based on the generated comparison result value. Further, the summing processing unit 240 allows the low-frequency summing result value to be compared with a predetermined threshold value, to generate a comparison result value, and allows a focusing location of the high-intensity ultrasound to be detected based on the generated comparison result value, through which the state and location of a lesion can be confirmed.

The comparing unit 250 compares the summing result value calculated by the summing processing unit 240 with a predetermined threshold value, to generate a comparison result value.

According to some embodiments, the comparing unit 250 compares a predetermined threshold value that is set based on the first echo signal with each summing result value calculated by the summing processing unit 240 for each region obtained by dividing the region of interest by the scanline in a time-division manner, to generate the comparison result value. The comparison result value means the difference between the summing result value and the predetermined threshold value, and when the comparison result value is larger than a predetermined value, it is determined that a cavitation is developed.

The mapping unit 260 detects development amount, development location, and the like of the cavitation by using the comparison result value generated by the comparing unit 250. More specifically, the mapping unit 260 divides the region of interest set on the target object by the scanline, divides each scanline in a time-division manner, and maps the comparison result value corresponding to each time-division region with reference to the time-division region, thus detecting the development location of the cavitation in the region of interest. In some embodiments, the development location of the cavitation is displayed with a different tone density depending on the magnitude of the comparison result value; however, the present disclosure is not limited to this scheme, but can be displayed with a numerical value or the like.

In some embodiments, the mapping unit 260 further confirms the development amount of the cavitation with time by using accumulated data that are accumulated in the summing processing unit 240.

Upon receiving a plurality of comparison result values for each frequency band from the comparison unit 250, the mapping unit 260 performs a plurality of mappings for each frequency band. For example, upon receiving the comparison result value for the high-frequency component and the comparison result value for the low-frequency component from the comparing unit 250, the mapping unit 260 divides the region of interest set on the target object by the scanline, divides each scanline in a time-division manner, and maps the comparison result value for the high-frequency component on each time-division region with reference to the time-division region, thus detecting the development location of the cavitation in the region of interest, and divides the region of interest set on the target object by the scanline, divides each scanline in a time-division manner, and maps the comparison result value for the low-frequency component on each time-division region with reference to the time-division region, thus detecting the focusing location of the high-intensity ultrasound, through which the state and location of a lesion can be confirmed.

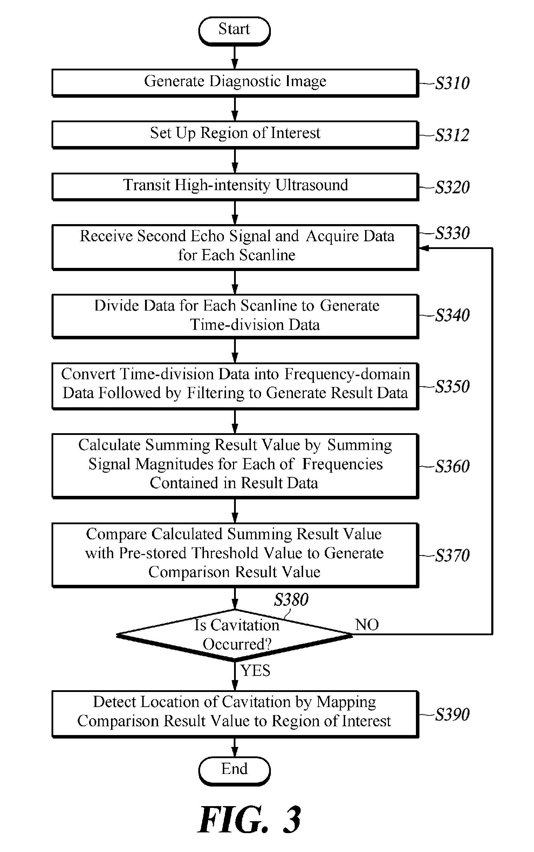

FIG. 3 is a flowchart of a method for detecting a cavitation in an ultrasound medical apparatus according to some embodiments.

The ultrasound medical apparatus 100 transmits the imaging ultrasound to the target object, receives the first echo signal reflected at the target object, and generates the diagnostic image based on the generated reception signal (Step S310).

The ultrasound medical apparatus 100 sets the region of interest within the diagnostic image through an operation or input from a user (Step S312), and transmits the high-intensity ultrasound to the focal-point spot set into the region of interest (Step S320).

The ultrasound medical apparatus 100 receives the second echo signal, and acquires the data for each scanline from the second echo signal (Step S330). The second echo signal means an echo signal obtained by receiving the imaging ultrasound and the high-intensity ultrasound reached the target object at the same time and reflected at the target object.

The ultrasound medical apparatus 100 divides the data for each scanline at predetermined regular time intervals, to generate a plurality of pieces of time-division data (Step S340).

The ultrasound medical apparatus 100 converts each piece of the time-division data among the plurality of pieces of time-division data generated at Step S340, and generates the result data by eliminating (filtering) the high-intensity frequency component from the frequency-domain data (Step S350).

The ultrasound medical apparatus 100 sums signal magnitudes by each frequency for each piece of result data, to calculate the summing result value (Step S360), divides the region of interest by the scanline, and compares the calculated summing result value with a predetermined threshold value for an area obtained by dividing each scanline in a time-division manner, so as to generate the comparison result value (Step S370).

The ultrasound medical apparatus 100 confirms the development of a cavitation based on the comparison result value (Step S380), and when it is determined that the cavitation is developed as a result of determination at Step S380, detects the location of the cavitation by mapping the comparison result value on the region of interest set at Step S312 (Step S390). That is, at Step S390, the ultrasound medical apparatus 100 divides the region of interest set on the target object by the scanline, divides each scanline in a time-division manner, and maps the comparison result value corresponding to each time-division region with reference to the time-division region, thus detecting the development location of the cavitation in the region of interest.

On the other hand, when Step S380 confirms that the cavitation is not developed, the ultrasound medical apparatus 100 performs an operation of acquiring the data at Step S330.

Although it is described that Steps S310 to S390 are sequentially performed in FIG. 3, it is a mere example of the technical idea of some embodiments of the present disclosure, accordingly one of ordinary skill would appreciate that various modification and alteration can be applied by modifying the sequence shown in FIG. 3 or performing one or more steps among Steps S310 to S390 in parallel, and hence FIG. 3 is not limited to the chronological order.

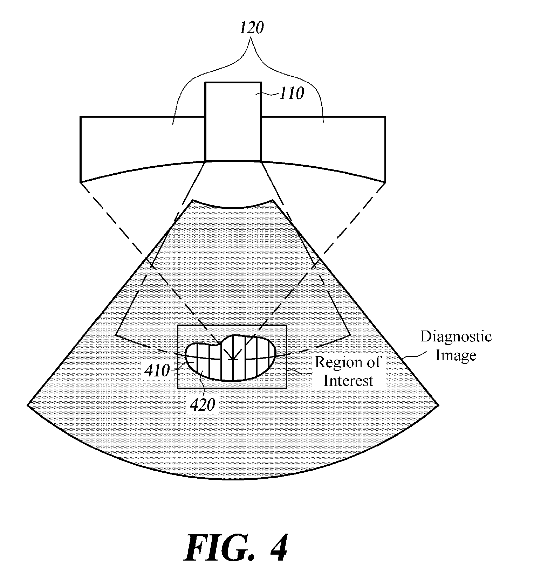

FIG. 4 is a schematic diagram for illustrating an operation of transmitting and receiving an imaging ultrasound and a treatment ultrasound by an ultrasound medical apparatus according to some embodiments.

The ultrasound medical apparatus 100 transmits the imaging ultrasound to the target object by using the imaging transducer 110, and receives the first echo signal reflected at the target object, to generate the diagnostic image.

The ultrasound medical apparatus 100 receives the location-of-interest information including coordinate values (x, y, z) from a user, in order to set the region of interest on the diagnostic image. In some embodiments, the region of interest is a region for treating cancer tissue, tumor tissue, legion tissue, or the like; however, the present disclosure is not limited to this scheme, but can be a region of square, circle, ellipse, or the like that is set based on the inputted location-of-interest information. The ultrasound medical apparatus 100 divides the region of interest into a first scanline 410, a second scanline 420, ..., and an nth scanline, to detect the cavitation.

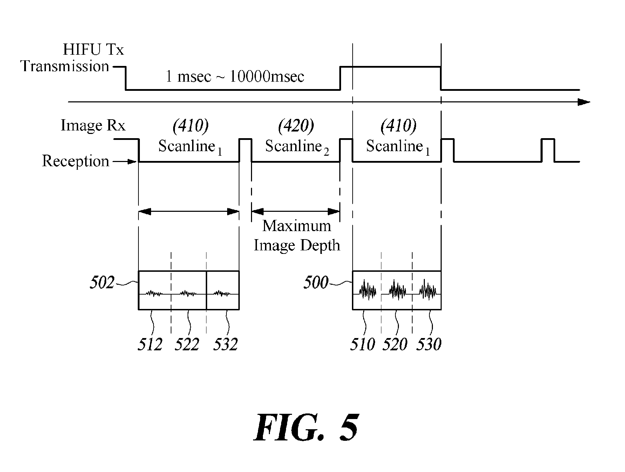

For example, the ultrasound medical apparatus 100 acquires data corresponding to the first scanline 410, and divides the data for the first scanline 410 by depths of the target object with regular intervals, i.e., at predetermined regular times, thus generating the result data for detecting the location of the cavitation. Details on this data processing are described with reference to FIG. 5.

FIG. 5 is a schematic diagram for illustrating a first echo signal and a second echo signal according to some embodiments.

As shown in FIG. 5, the ultrasound medical apparatus 100 transmits the image ultrasound with no high-intensity ultrasound transmitted, and acquires first scanline data 502 for the first scanline 410 by using the first echo signal reflected at the region of interest. The ultrasound medical apparatus 100 divides the first scanline data 502 at regular time intervals, to generate a plurality of pieces of threshold data 512, 522, and 532, and converts each piece of threshold data into frequency-domain data, to calculate a threshold value by summing signal magnitudes by each frequency for each piece of threshold data.

Further, the ultrasound medical apparatus 100 acquires second scanline data 500 for the first scanline 410 by using the second echo signal reflected when the high-intensity ultrasound is transmitted to the focal-point spot. The ultrasound medical apparatus 100 divides the second scanline data 500 at the same time intervals as that for the first scanline data 502, to generate a plurality of time-division data 510, 520, and 530, and converts each piece of time-division data into frequency-domain data, to calculate summing result value for each piece of time-division data.

The ultrasound medical apparatus 100 compares the threshold value of the first scanline data 502 with the summing result value of the second scanline data 500 corresponding to the same region, to calculate a comparison value. For example, the ultrasound medical apparatus 100 compares the threshold value for the threshold data 512 of the first scanline data 502 with the summing result value for the time-division data 510 of the second scanline data 500, to calculate the comparison value for the same location of the first scanline 410. Thereafter, the ultrasound medical apparatus 100 confirms the development of the cavitation at the first scanline 410 by using the comparison value, and maps the comparison value on the region of interest, to detect the development location of the cavitation.

It is described that the ultrasound medical apparatus 100 shown in FIG. 5 detects the development amount and the development location of the cavitation at a single scanline in the region of interest, but the same goes for a plurality of scanlines, to detect the development amount and the development location of the cavitation at the plurality of scanlines.

FIG. 6 is a graph showing an operation of processing data for detecting a cavitation in an ultrasound medical apparatus according to some embodiments.

The graph shown in FIG. 6 indicates the result data generated by dividing the region of interest set on the target object by each scanline, dividing each scanline in a time-division manner, and processing data for a single region among the time-division regions.

Shown in FIG. 6 at (a) is a graph of the time-division data obtained by dividing the data for each scanline acquired from the second echo signal by the ultrasound medical apparatus 100 by a predetermined time, the graph at (b) shows the frequency-domain data obtained by converting the time-division data shown at (a) by using the FFT, and the graph at (c) is the result data obtained by eliminating (filtering) the high-intensity frequency component from the graph shown at (b). Shown in FIG. 6 at (d) is a schematic diagram of the location where the cavitation is developed, based on the frequency component included in the result data.

The ultrasound medical apparatus 100 divides the region of interest set on the target object by each scanline, and converts a piece of data 510 among a plurality of time-division data obtained by dividing the scanline data shown in FIG. 6 (a) acquired from the second echo signal for the scanline into the frequency-domain data shown in FIG. 6 (b) by using the FFT. The ultrasound medical apparatus 100 generates the result data shown in FIG. 6 (c) with the high-intensity frequency component corresponding to the high-intensity ultrasound eliminated by filtering the frequency-domain data with a filter having a sawtooth-shaped window.

The ultrasound medical apparatus 100 sums the signal magnitudes by each frequency for each piece of result data, to calculate the summing result value, divides the region of interest set on the target object, divides each scanline in a time-division manner, and maps the comparison result value obtained by comparing the calculated summing result value with a predetermined threshold value on each piece of time-division region with reference to the time-division region, to detect the development location of the cavitation in the region of interest, as shown in FIG. 6 (d). In some embodiments, the development location of the cavitation is displayed with a different tone density depending on the magnitude of the comparison result value; however, the present disclosure is not limited to this scheme, but can be displayed with a numerical value or the like.

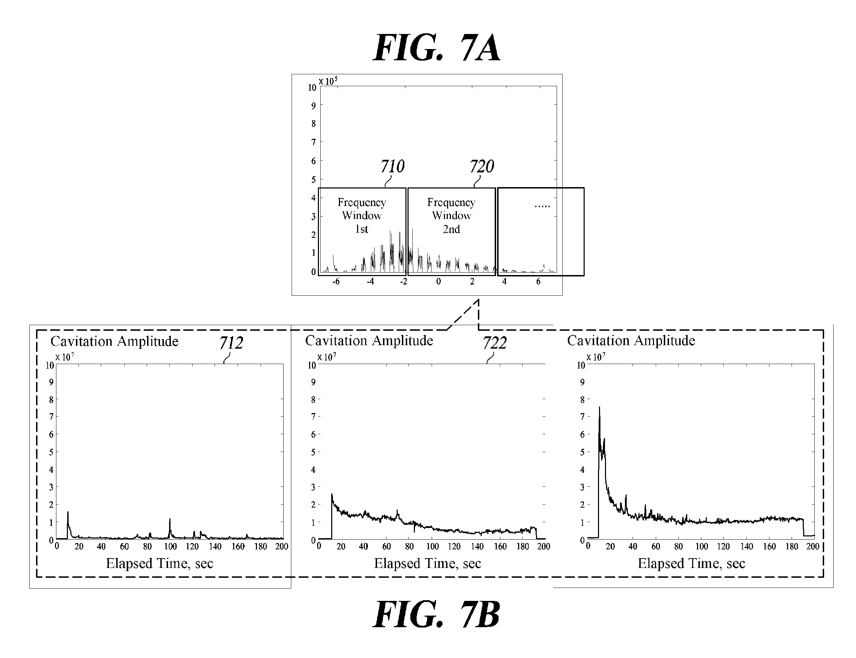

FIG. 7 is a graph showing an operation of dividing result data by magnitude and calculating respective summing result values in an ultrasound medical apparatus according to some embodiments.

Shown in FIG. 7 at (a) is a graph same as the result data graph shown in FIG. 6 (c), and as a step of generating the result data is described in FIG. 6, a description of the same step is omitted.

FIG. 7 (a) is a graph showing the result data generated by dividing the region of interest set on the target object by each scanline, dividing each scanline in a time-division manner, and processing data for a single region among the time-division regions.

As shown in FIG. 7 (a), the ultrasound medical apparatus 100 divides the result data based on the frequency band into first band data 710, second band data 720, ..., and nth band data. The ultrasound medical apparatus 100 sums signal magnitudes for each frequency included in each piece of band data divided based on the frequency band, to calculate summing result value. The ultrasound medical apparatus 100 generates summing result data 712 and 722 for the region of interest shown in FIG. 7 (b) by using the summing result value calculated from the result data for each of the time-division regions. For example, the ultrasound medical apparatus 100 generates first summing result data 712 for the region of interest by using summing result data obtained by summing low-frequency signal magnitudes corresponding to a low-band frequency such as the first band data 710. The ultrasound medical apparatus 100 can detect the focusing location of the high-intensity ultrasound by using the first summing result data 712, through which the state and location of a lesion can be confirmed.

On the other hand, the ultrasound medical apparatus 100 can generate second summing result data 722 for the region of interest by summing high-frequency signal amplitudes corresponding to a high band frequency such as second band data 720 and accumulating the obtained summing results. The ultrasound medical apparatus 100 can confirm a development amount of the cavitation by using the second summing result data 722 and detect the development location of the cavitation by mapping the second summing result data 722 on the region of interest. When detecting the development location of the cavitation by using only the high band frequency, the ultrasound medical apparatus 100 can shorten the detection time owing to the reduced amount of the data to calculate.

Although exemplary embodiments of the present disclosure have been described for illustrative purposes, those skilled in the art will appreciate that various modifications, additions and substitutions are possible, without departing from the spirit and scope of the claimed invention. Therefore, exemplary embodiments of the present disclosure have been described for the sake of brevity and clarity. Accordingly, one of ordinary skill would understand the scope of the claimed invention is not to be limited by the explicitly described above embodiments but by the claims and equivalents thereof.

### CROSS-REFERENCE TO RELATED APPLICATION

If applicable, this application claims priority under 35 U.S.C §119(a) of Patent Application No. 10-2013-0021951, filed on February 28, 2013 in Korea, the entire content of which is incorporated herein by reference. In addition, this non-provisional application claims priority in countries, other than the U.S., with the same reason based on the Korean patent application, the entire content of which is hereby incorporated by reference.

## Claims

1. An ultrasound medical apparatus, comprising:
an imaging transducer configured
to transmit an imaging ultrasound to a target object, and
to receive a first echo signal reflected from the target object;
a treatment transducer configured to transmit a high-intensity ultrasound to a focal-point spot on the target object;
a data processing unit configured
to divide data for each scanline acquired from a second echo signal reflected at the focal-point spot at predetermined regular time intervals, to generate time-division data,
to convert the time-division data into frequency domain data, and
to generate result data from the frequency domain data; and
a detecting unit configured to detect a location of a cavitation based on a frequency component included in each piece of the result data.

2. The ultrasound medical apparatus according to claim 1, wherein the detecting unit includes
a summing processing unit configured to sum signal magnitudes by each frequency for each piece of the result data, to generate a summing result value,
a comparing unit configured to compare a predetermined threshold value that is set based on the first echo signal with the summing result value, to generate a comparison result value; and
a mapping unit configured to map the comparison result value on a region of interest, to detect the location of the cavitation.

3. The ultrasound medical apparatus according to claim 2, wherein the summing processing unit is configured
to divide the frequency component included in each of the result data by each frequency band, and
to calculate the summing result value for each frequency band.

4. The ultrasound medical apparatus according to claim 3, wherein the summing processing unit is configured to generate a high-frequency summing result value obtained by summing high-frequency signal magnitudes among frequency components included in each piece of the result data or a low-frequency summing result value obtained by summing low-frequency signal magnitudes among the frequency components included in each of the result data, as the summing result value.

5. The ultrasound medical apparatus according to claim 4, wherein
the comparing unit is configured to compare the high-frequency summing result value with the predetermined threshold value, to generate a high-frequency comparison result value, and
the mapping unit is configured to map the high-frequency comparison result value on the region of interest, to detect the location of the cavitation.

6. The ultrasound medical apparatus according to claim 4, wherein
the comparing unit is configured to compare the low-frequency summing result value with the predetermined threshold value, to generate a low-frequency comparison result value, and
the mapping unit is configured to map the low-frequency comparison result value on the region of interest, to detect the location of the cavitation.

7. The ultrasound medical apparatus according to claim 2, wherein the mapping unit is configured to confirm a development amount of the cavitation with time by using accumulated data of the summing result value.

8. The ultrasound medical apparatus according to claim 1, wherein the data processing unit includes
a data acquiring unit configured
to divide the imaging ultrasound by each scanline for transmission, and
to acquire the data for each scanline for each of scanlines,
a data converting unit configured
to divide the data for each scanline by the predetermined time interval, to generate the time-division data, and
to convert the time-division data in a time domain into frequency-domain data by using a frequency analysis; and
a filter processing unit configured filter a high-intensity frequency component included in the frequency-domain data, to generate the result data.

9. The ultrasound medical apparatus according to claim 8, wherein the data converting unit is configured to convert the time-division data into the frequency-domain data by using a Fast Fourier Transform (FFT).

10. The ultrasound medical apparatus according to claim 8, wherein the filter processing unit is configured filter at least one frequency component of a main frequency component and a harmonic frequency component by using a filter having a sawtooth-shaped window from the frequency-domain data, to generate the result data.

11. The ultrasound medical apparatus according to claim 1, further comprising a synchronizing unit configured to synchronize transmissions of the imaging ultrasound and the high-intensity ultrasound to render the imaging ultrasound and the high-intensity ultrasound reach the focal-point spot at the same time.

12. The ultrasound medical apparatus according to claim 1, further comprising:
an image processing unit configured
to generate a reception signal by using the first echo signal,
to allow a diagnostic image to be formed based on the reception signal, and
to allow the diagnostic image to be outputted via a display unit; and
a user input unit configured
to receive location-of-interest information for setting a region of interest on the diagnostic image, and
to receive focal-point information for setting a focal-point spot within the region of interest.

13. A method for detecting a cavitation by an ultrasound medical apparatus, the method comprising:
a first echo signal receiving step including:
transmitting an imaging ultrasound to a target object, and
receiving a first echo signal reflected from the target object;
a high-intensity ultrasound transmitting step including transmitting a high-intensity ultrasound to a focal-point spot on the target object;
a data processing step including:
dividing scanline data acquired from a second echo signal reflected from the focal-point spot in a time-division manner and generating time-division data;
converting the time-division data into frequency domain data, and
generating result data from the frequency domain data; and
a detecting step including detecting a location of a cavitation based on a frequency component included in the result data.

14. The method according to claim 13, wherein the detecting step includes
summing processing including summing signal magnitudes by each frequency for each piece of the result data and generating a summing result value,
comparing processing including comparing a predetermined threshold value that is set based on the first echo signal with the summing result value and generating a comparison result value; and
mapping processing including mapping the comparison result value on a region of interest and detecting the location of the cavitation.

15. The method according to claim 13, wherein the data processing step includes
a data acquiring step including
dividing the imaging ultrasound by each scanline for transmission, and
acquiring the data for each scanline for each of scanlines,
a data converting step including
dividing the data for each scanline by the predetermined time interval, to generate the time-division data, and
converting the time-division data in a time domain into frequency-domain data by using a frequency analysis; and
a filter processing step including
filtering a high-intensity frequency component included in the frequency-domain data, and
generating the result data.

16. The method according to claim 13, further comprising:
an image processing step including
generating a reception signal by using the first echo signal, and
allowing a diagnostic image to be formed based on the reception signal; and
a focal-point spot setting step including
receiving location-of-interest information for setting a region of interest on the diagnostic image,
setting the region of interest based on the location-of-interest information,
receiving focal-point information for setting a focal-point spot, and
setting the focal-point spot within the region of interest based on the focal-point information.
